# EUROPEAN PATENT APPLICATION

(11) **EP 4 609 883 A1**
(43) Date of publication of application: **03.09.2025**
(21) Application number: 22963227.8
(22) Date of filing: 04.11.2022
(51) Int. Cl.: A61L 9/22, F24F 8/80, F24F 8/24

(54) **ROUND HOLE NEEDLE-LEVEL AIR CORONA DISINFECTION DEVICE**

(30) Priority: 25.10.2022 CN 202222814971 U
(71) Applicant: We Air Health (Shanghai) Technology Co., Ltd, Shanghai 200436 (CN)
(72) Inventor: ZHANG, Ming, Shanghai 200436 (CN)
(74) Representative: Maiwald GmbH
(86) International application number: PCT/CN2022/129749
(87) International publication number: WO 2024/087246

(57) **Abstract**

A round hole needle-level air corona disinfection device, relating to the technical field of air disinfection, and comprising a high-voltage pack (2), a negative electrode plate (3), and a positive electrode support strip (5). The positive electrode support strip (5) is provided with a plurality of insulating connection columns (6) at intervals; a plurality of metal round holes (4) are provided on the negative electrode plate (3); the interior of each metal round hole (4) is divided into an air inlet (7) located at the top, a corona area (9) located at the middle, and an air outlet (8) located at the bottom; each discharge alloy needle (1) has one end fixedly connected to the interior of the positive electrode support strip (5), and the other end located at the axis of the metal round hole (4), lower than the air inlet (7), and higher than the air outlet (8). The tip of the discharge alloy needle (1) discharges in the middle of the axis of the metal round hole (4) to generate the corona area (9), so that the air flowing from the top of the metal round hole (4) to the bottom of the metal round hole (4) is subjected to strong oxidation disinfection, thereby improving the air disinfection capacity; moreover, free radicals and positive ions released from the tip of the discharge alloy needle (1) enable a large number of positive and negative ions to agglomerate on particles, thereby effectively reducing PM2.5 in the air.

## Description

### FIELD

The present disclosure belongs to a field of air disinfection technologies, in particular to a circular hole and needle based air corona disinfection device.

### BACKGROUND

After COVID-19, the airborne epidemic virus has become the focus of everyone's attention, the source of infection of most airborne epidemics is the infected patient, and the mode of transmission is that it spreads from person to person when there are people around. Thus, it is particularly important to realize air disinfection in a manned environment.

The existing electrostatic precipitator technology mainly relies on adsorption to realize dust removal and purification. A kind of electrostatic technology based on a cylindrical tube and a needle effectively solves the problem that the ozone level exceeds the standard, and uses the corona field generated by a tip of the needle to realize air disinfection, and also, a wall of the tube absorbs the charged particulate matter after passing through the corona zone. However, in the process of realizing the technical scheme in the embodiments of the present disclosure, the inventor of the present disclosure finds that the above technology has at least the following technical problems.

The ozone level of the traditional electrostatic precipitator technology exceeds the standard, and the disinfection ability of the traditional electrostatic precipitator technology is limited. Although the cylindrical tube and needle based technology solves the problem that the ozone level exceeds the standard and produces the corona field to enhance the disinfection ability, the ability of the tube wall to remove dust by absorption is limited. Moreover, the thickness of the whole equipment is also limited, which is generally greater than 2.5 cm, while the traditional filter screen generally has a thickness between 1.5 centimeters and 2.4 centimeters. Thus, this technology cannot directly replace the traditional filter screen (due to lack of installation space).

### SUMMARY

### TECHNICAL PROBLEM

### SOLUTION TO PROBLEM

### BENIFICAL EFFECTS OF THE PRESENT DISCLOSURE

### BENIFICAL EFFECTS

In order to overcome the existing shortcomings, embodiments of the present disclosure provide a circular hole and needle based air corona disinfection device. A cylindrical tube is removed, a circular hole is directly formed in a negative metal plate, and a tip of a discharge alloy needle is used to discharge to generate a corona zone in the middle of the metal-surrounded circular hole at an axis of the metal-surrounded circular hole, so that the air flowing from the top of the metal-surrounded circular hole to the bottom of the metal-surrounded circular hole is subjected to strong oxidative disinfection, which solves the problems that the ozone level in the existing electrostatic precipitator technology exceeds the standard and the disinfection capacity of the existing electrostatic precipitator technology is limited, and also solves the problems that the cylindrical hole and needle based electrostatic technology has a large thickness and the maintenance of the cylindrical tube is troublesome.

The technical scheme adopted by the embodiments of the present disclosure to solve the technical problems is as follows.

A circular hole and needle based air corona disinfection device includes three parts, namely, a high-voltage package configured to provide a power set-up, a negative plate with a metal-surrounded circular hole and a positive bracket.

The negative plate is connected with a negative electrode of the high-voltage package through a wire, a plurality of metal-surrounded circular holes are formed in the negative plate, and a discharge alloy needle is arranged in each metal-surrounded circular hole.

The positive bracket is connected with a positive electrode of the high-voltage package through a wire, one end of the discharge alloy needle is fixedly connected inside the positive bracket, and the other end of the discharge alloy needle is located at a central axis of the metal-surrounded circular hole.

In a possible implementation, a voltage of the high-voltage package is controlled to be between 4000 volts and 8000 volts, and a current of the high-voltage package is controlled to be between 500 microamperes and 1 milliampere.

In a possible implementation, an axis of the metal-surrounded circular hole overlaps with an axis of the discharge alloy needle.

In a possible implementation, an interior of the positive bracket is in a grid shape.

In a possible implementation, the positive bracket is provided with a plurality of insulating connection columns arranged at intervals, and the negative plate is fixedly connected with the positive bracket through insulating columns.

In a possible implementation, a distance between the discharge alloy needle and an air inlet of the metal-surrounded circular hole is 1-2 millimeters.

In a possible implementation, an interior of the metal-surrounded circular hole is divided into an air inlet at a top of the metal-surrounded circular hole, a corona zone in the middle of the metal-surrounded circular hole and an air outlet at a bottom of the metal-surrounded circular hole.

In a possible implementation, a radius of the metal-surrounded circular hole is 1 centimeters to 1.5 centimeters, and a length of the discharge alloy needle is 1.2-1.5 times of the radius of the circular hole.

The present disclosure has the following beneficial effects.

First, in this scheme, the tip of the discharge alloy needle is used to discharge to generate the corona zone in the middle of the metal-surrounded circular hole at the axis of the metal-surrounded circular hole, so that the corona field exerts strong oxidative disinfection on the air flowing from the top of the metal-surrounded circular hole to the bottom of the metal-surrounded circular hole.

Second, in this scheme, by using free radicals and positive ions released from the tip of the discharge alloy needle, a large number of positive and negative ions will coagulate with the particulate matter, thus effectively reducing the PM2.5 in the air, and the adsorption part of the traditional cylindrical tube is removed, thus saving the precious physical space, facilitating the direct replacement of the traditional filter screen, and reducing the difficulty of maintaining the cylindrical tube in the later stage.

### BRIEF DESCRIPTION OF THE DRAWINGS

### DESCRIPTION OF THE DRAWINGS

Fig. 1 is a schematic view of an overall structure of the present disclosure.
Fig. 2 is an enlarged view of part A in Fig. 1 of the present disclosure.
Fig. 3 is a front view of the present disclosure.
Fig. 4 is an exploded view of the present disclosure.
Fig. 5 is an enlarged view of part B in Fig. 4 of the present disclosure.
Fig. 6 is a sectional view of a negative plate of the present disclosure.

Reference signs: 1 discharge alloy needle; 2 high-voltage package; 3 negative plate; 4 metal-surrounded circular hole; 5 positive bracket; 6 insulating connection column; 7 air inlet; 8 air outlet; 9 corona zone.

### IMPLEMENTATION OF BEST EMBODIMENTS OF THE PRESENT DISCLOSURE BEST EMBODIMENTS OF THE PRESENT DISCLOSURE

In a circular hole and needle based air corona disinfection device provided by embodiments of the present disclosure, a cylindrical tube is removed, a circular hole is directly formed in a negative metal plate, and a tip of a discharge alloy needle is used to discharge in the middle of the metal-surrounded circular hole at an axis of the metal-surrounded circular hole to generate a corona zone, so that the air flowing from the top of the metal-surrounded circular hole to the bottom of the metal-surrounded circular hole is subjected to strong oxidative disinfection.

In order to solve the above problems in the related art, the technical scheme in the embodiments of the present disclosure has the following general idea.

### Embodiment 1

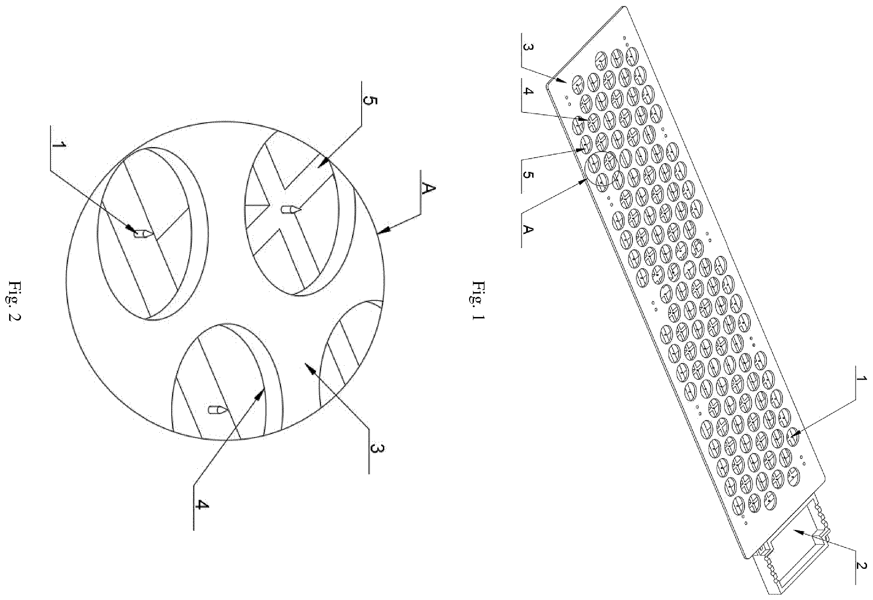

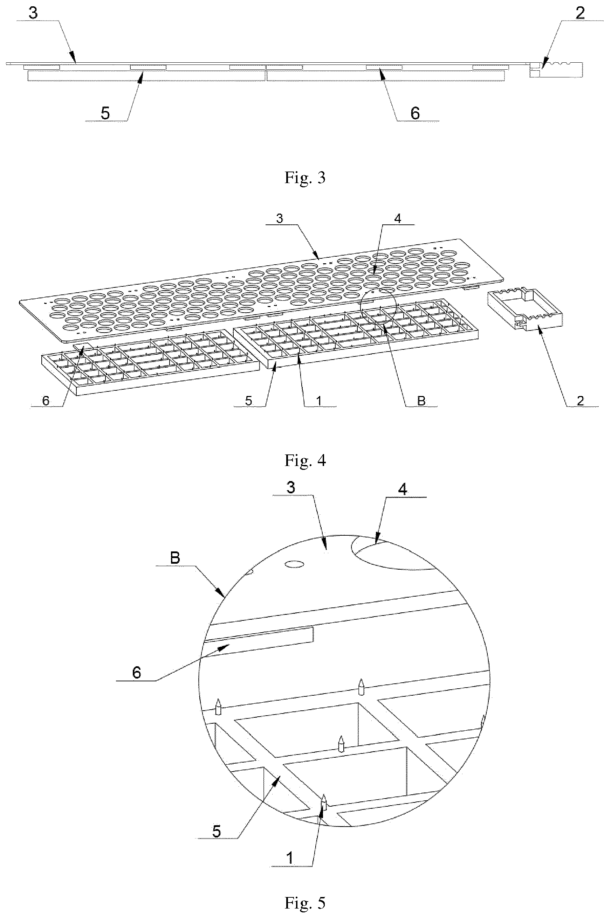

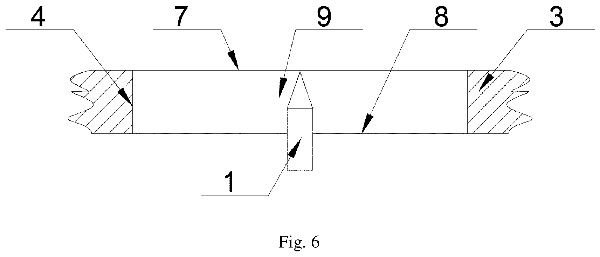

This embodiment introduces the specific structure of a circular hole and needle based air corona disinfection device, as shown in Figs. 1-6, and the device includes a high-voltage package 2, a negative plate 3, and a positive bracket (e.g., a positive strip) 5.

The high-voltage package 2 is configured to provide a voltage.

In order to ensure human safety, the voltage of the high-voltage package 2 is controlled to be between 4000 volts and 8000 volts, and a current of the high-voltage package 2 is controlled to be between 500 microamperes and 1 milliampere, and also is limited at 1 milliampere to ensure the safety of human touch.

The negative plate 3 is connected with a negative electrode of the high-voltage package 2 through a wire, and defines a plurality of metal-surrounded circular holes 4, and a discharge alloy needle 1 is arranged in each metal-surrounded circular hole 4.

A tip of the discharge alloy needle 1 discharges generate a large number of free radicals and positive ions, thus exerting a strong oxidative disinfection effect on the air passing through the metal-surrounded circular hole 4.

The positive bracket 5 is connected with a positive electrode of the high-voltage package 2 through a wire. One end of the discharge alloy needle 1 is fixedly connected inside the positive bracket 5, and the other end of the discharge alloy needle 1 is located at a central axis of the metal-surrounded circular hole 4.

The discharge alloy needle 1 is fitted with a closed area defined by the metal-surrounded circular hole 4 formed in the negative plate 3. When the tip of the discharge alloy needle 1 discharges, a corona zone 9 which rotates at a high speed is formed in the middle of the metal-surrounded circular hole 4, thus effectively increasing the disinfection path of viruses and bacteria and improving the disinfection efficiency.

In some examples, an axis of the metal-surrounded circular hole 4 overlaps with an axis of the discharge alloy needle 1, so that the discharge alloy needle 1 is located at the corresponding central axis of the metal-surrounded circular hole 4, so as to ensure the uniform discharge of the tip of the needle and limit the generation of ozone, and the discharge alloy needle 1 is fitted with the metal-surrounded circular hole 4 to form the effective corona zone 9 in the middle of the metal-surrounded circular hole 4.

In some examples, the positive bracket 5 is provided with a plurality of insulating connection columns 6 arranged at intervals, the negative plate 3 and the positive bracket 5 remain parallel to each other, and the insulating connection columns 6 are connected between the negative plate 3 and the positive bracket 5, so that the interval between the negative plate 3 and the positive bracket 5 is kept equal to or larger than 10 millimeters, thus realizing air insulation.

By adopting the above technical scheme:
in the above design, the tip of the discharge alloy needle 1 is used to discharge in the middle of the metal-surrounded circular hole 4 at the axis of the metal-surrounded circular hole 4, so that the corona zone 9 may be generated, and thus the air disinfection may be realized in a manned environment. The corona zone 9 which rotates at the high speed is formed at an ionization area in the metal-surrounded circular hole 4 by using the tip of the discharge alloy needle 1 and the high-voltage ionization technology, so that the air flowing from the top of the metal-surrounded circular hole 4 to the bottom of the metal-surrounded circular hole 4 may be subjected to strong oxidative disinfection.

### Embodiment 2

Based on Embodiment 1, this embodiment introduces the specific structure of the circular hole and needle based air corona disinfection device. The device of this embodiment includes a high-pressure package 2, a positive bracket 5, a metal-surrounded circular hole 4 formed in the positive bracket 5, a discharge alloy needle 1 located in the metal-surrounded circular hole 4 and at a center of the metal-surrounded circular hole 4, and an insulating connecting column 6. The metal-surrounded circular hole 4 has an air inlet 7 at a top and an air outlet 8 at a bottom, and the air flows through the metal-surrounded circular hole 4 towards the positive bracket 5.

A positive electrode of the high-voltage package 2 is connected with the positive bracket 5 through a wire, and one end of the discharge alloy needle 1 is fixed on the positive bracket 5. The discharge alloy needle 1 is oriented upwards vertically so that an axis of the discharge alloy needle 1 overlaps with an axis of the metal-surrounded circular hole 4, and the other end of the discharge alloy needle 1 is 1-2 millimeters away from the air inlet of the metal-surrounded circular hole 4. A negative electrode of the high-voltage package 2 is connected with a negative plate 3 through a wire, and a plurality of insulating connecting columns 6 are installed between the negative plate 3 and the positive bracket 5 so that the negative plate 3 and the positive bracket 5 remain parallel to each other and are spaced apart by a distance equal to or larger than 10 millimeters, thus realizing air insulation.

In the process of specific use, the user's operation steps are as follows.

In step S1, the high-voltage package 2 with a voltage of 4000-8000 volts and a current of 500 microamperes to 1 milliampere is used to input a current into the discharge alloy needle 1 via the positive bracket 5 through a wire.

In step S2, the tip of the discharge alloy needle 1 discharges to generate a large number of free radicals and positive ions, and form the corona zone 9 which rotates at the high speed in the metal-surrounded circular hole 4, so that the air is subjected to the strong oxidative disinfection.

In step S3, the positive ions and free radicals released by the tip of the discharge alloy needle 1 inside the metal-surrounded circular hole 4 exert the strong oxidative disinfection on the virus flowing through the metal-surrounded circular hole 4, and also, a large number of positive and negative ions will coagulate with the particulate matter, thus effectively reducing PM2.5 in the air.

By adopting the above technical scheme:
in the above design, a large number of free radicals and positive ions are generated by discharging the current from the tip of the discharge alloy needle 1 by means of the high-voltage package 2. When the free radicals and positive ions exert the strong oxidative disinfection on the virus flowing through the metal-surrounded circular hole 4, a large number of positive and negative ions will coagulate with the particulate matter, thus effectively reducing PM2.5 in the air. Therefore, on the basis of inhibiting ozone generation, the corona zone formed by the tip of the alloy needle 1 in the metal-surrounded circular hole 4 is used for efficient air disinfection, and thus the adsorption part of the traditional cylindrical tube is removed, which saves the precious physical space, realizes an ultra-thin structure of 1.5-2 centimeters, facilitates the direct replacement of the traditional filter screen, and also reduces the maintenance work of the cylindrical tube.

Finally, it should be noted that obviously, the above embodiments are only examples for clearly explaining the present disclosure, and they are not limitations to the implementations. For those skilled in the art, other changes or modifications in different forms may be made on the basis of the above description. It is not necessary and impossible to exhaust all the embodiments here. However, the obvious changes or modifications derived therefrom are still within the protection scope of the present disclosure.

## Claims

1. A circular hole and needle based air corona disinfection device, comprising:
a high-voltage package (2) configured to provide a voltage;
a negative plate (3) connected with a negative electrode of the high-voltage package (2) through a wire, and defining a plurality of metal-surrounded circular holes (4), wherein a discharge alloy needle (1) is arranged in each metal-surrounded circular hole (4); and
a positive bracket (5) connected with a positive electrode of the high-voltage package (2) through a wire, wherein an end of the discharge alloy needle (1) is fixedly connected inside the positive bracket (5).

2. The circular hole and needle based air corona disinfection device according to claim 1, wherein a voltage of the high-voltage package (2) is controlled to be between 4000 volts and 8000 volts, and a current of the high-voltage package (2) is controlled to be between 500 microamperes and 1 milliampere.

3. The circular hole and needle based air corona disinfection device according to claim 1, wherein an axis of the metal-surrounded circular hole (4) overlaps with an axis of the discharge alloy needle (1).

4. The circular hole and needle based air corona disinfection device according to claim 1, wherein an interior of the positive bracket (5) is in a grid shape.

5. The circular hole and needle based air corona disinfection device according to claim 1, wherein the positive bracket (5) is provided with a plurality of insulating connection columns (6) arranged at intervals, and the negative plate (3) is fixedly connected with the positive bracket (5) through the insulating connection columns (6).

6. The circular hole and needle based air corona disinfection device according to claim 1, wherein a distance between a tip of the discharge alloy needle (1) and an air inlet of the negative plate (3) is 1-2 millimeters.

7. The circular hole and needle based air corona disinfection device according to claim 1, wherein an interior of the metal-surrounded circular hole (4) is divided into an air inlet (7) at a top of the metal-surrounded circular hole (4), a corona zone (9) in the middle of the metal-surrounded circular hole (4) and an air outlet (8) at a bottom of the metal-surrounded circular hole (4).

8. The circular hole and needle based air corona disinfection device according to claim 1, wherein a radius of the metal-surrounded circular hole (4) is 1 centimeters to 1.5 centimeters, and a length of the discharge alloy needle (1) is 1.2-1.5 times of the radius of the circular hole.
